# EUROPÄISCHE PATENTSCHRIFT

(11) **EP 4 520 389 B1**
(45) Veröffentlichungstag und Bekanntmachung des Hinweises auf die Patenterteilung: **04.02.2026**
(21) Anmeldenummer: 24196987.2
(22) Anmeldetag: 28.08.2024
(51) Int. Cl.: A61N 1/05

(54) **VORRICHTUNG ZUR NERVENSTIMULATION**
APPARATUS FOR NERVE STIMULATION
DISPOSITIF DE STIMULATION NERVEUSE

(30) Priorität: 05.09.2023 DE 102023123896
(43) Veröffentlichungstag der Anmeldung: 12.03.2025
(73) Patentinhaber: Pajunk GmbH Medizintechnologie, 78187 Geisingen (DE)
(72) Erfinder: PAJUNK-SCHELLING, Simone, 78187 Geisingen (DE); HAUGER, Martin, 78166 Donaueschingen (DE)
(74) Vertreter: Westphal, Mussgnug & Partner Patentanwälte mbB

(56) Entgegenhaltungen:
- WO-A1-97/15347
- DE-U1- 29 820 525
- US-A- 4 824 433
- US-A1- 2008 114 432
- US-A1- 2011 054 383

## Beschreibung

Die Erfindung betrifft eine Vorrichtung zur Nervenstimulation.

Es ist bekannt, zur Schmerzlinderung oder zur Lösung von muskulären Verspannungen elektrische Strompulse, beispielsweise niedrigfrequenten Wechselstrom, mit niedriger Spannung anhand von auf der Haut platzierten Elektroden zu übertragen, um auf diese Weise transkutan die Nerven zu stimulieren. Dabei stellt die Haut jedoch einen vergleichsweise großen Übergangswiderstand dar. Zudem erfolgt die Stimulation in der Regel großflächig.

Vorrichtungen zur Nervenstimulation sind beispielsweise aus der WO 97/15347 A1, der US 2011/054383 A1, der US 2008/114432 A1 und der US 4 824 433 A bekannt. Die DE 298 20 525 U1 offenbart eine Vorrichtung zur Nervenstimulation mit den Merkmalen des Oberbegriffs des Patentanspruchs 1.

Es ist daher Aufgabe der Erfindung, eine verbesserte Vorrichtung zur Nervenstimulation anzugeben, welche insbesondere vielseitig einsetzbar ist und mit welcher vorzugsweise eine direktere Stimulation möglich ist.

Die Aufgabe der Erfindung wird gelöst durch eine Vorrichtung zur Nervenstimulation mit den Merkmalen des Patentanspruchs 1.

Vorteilhafte Ausgestaltungen und Weiterbildungen der Erfindung sind in den abhängigen Ansprüchen angegeben.

Die erfindungsgemäße Vorrichtung zur Nervenstimulation umfasst einen elektrisch leitenden Stimulationsdraht mit einem Durchmesser, einem distalen Ende und einem proximalen Ende, welcher in einem elektrisch isolierenden Schlauch mit einem Innendurchmesser, einem Außendurchmesser, einem distalen Ende und einem proximalen Ende angeordnet ist, wobei der Innendurchmesser des Schlauchs größer ist als der Durchmesser des Stimulationsdrahts, so dass ein Spalt zur Zufuhr von Fluid in dem Schlauch gebildet ist, wobei an dem distalen Ende des Stimulationsdrahts eine Stimulationselektrode elektrisch leitend angeordnet ist, welche über das distale Ende des Schlauchs vorsteht, und wobei das proximale Ende des Stimulationsdrahts über das proximale Ende des Schlauchs vorsteht und um das proximale Ende des Schlauchs umgebogen auf die Außenseite des Schlauchs geführt ist, wobei ein elektrisch leitendes Stimulationskabel an dem proximalen Ende des Stimulationsdrahts elektrisch leitend verbunden ist und wobei ein Zuspritzschlauch abschnittsweise über das proximale Ende des Schlauchs abdichtend gestülpt ist.

Die erfindungsgemäße Vorrichtung eignet sich sowohl zur Nervenstimulation als auch zur Zufuhr eines Fluids, beispielsweise einer Flüssigkeit wie eines Anästhetikums. Dadurch, dass der Stimulationsdraht innerhalb des Lumens des Schlauchs angeordnet ist, ergibt sich erfindungsgemäß ein besonders kompakter Aufbau. Der am proximalen Ende des Schlauchs umgebogene Stimulationsdraht ermöglicht eine einfache Anbindung des Stimulationskabels auf der Außenseite des Schlauchs, ohne den Stimulationsdraht durch die Wandung des Schlauchs führen zu müssen, so dass eine mögliche Undichtigkeitsstelle vermieden werden kann.

Aufgrund der Flexibilität des Schlauchs kann zudem eine langgestreckte, flexible Vorrichtung bereitgestellt werden, die entweder durch Kanülen oder Verweilkanülen in den Körper, aber auch durch eine natürliche Körperöffnung, beispielsweise die Nase, die Blase oder die Vagina, in den Körper ohne das Erfordernis, einen Schnitt in die Haut setzen zu müssen, eingeführt werden kann. Dadurch kann eine Stimulation auch von Nerven erfolgen, die durch außen am Körper auf die Haut geklebte Stimulationselektroden nur schwer oder gar nicht zugänglich sind.

Gemäß einer vorteilhaften Weiterbildung der Erfindung weist die Stimulationselektrode eine abgerundete Außenfläche auf und ist beispielsweise kalottenförmig, kugelförmig, ellipsoidförmig, pilzförmig, birnenförmig oder eiförmig ausgebildet. Durch die abgerundete Ausgestaltung können Verletzungen am Körper der zu behandelnden Person vermieden werden. Zudem kann eine derartige Ausgestaltung der Stimulationselektrode das Vorschieben der Vorrichtung, insbesondere in die Körperöffnung, vereinfachen.

Eine bevorzugte Ausführungsform der Erfindung sieht vor, dass die Stimulationselektrode zumindest in einem Querschnitt einen Außendurchmesser aufweist, welcher größer ist als der Außendurchmesser des Schlauchs. Mit anderen Worten ist zumindest der größte Außendurchmesser der Stimulationselektrode größer als der Außendurchmesser des Schlauchs. Durch eine derartige Ausgestaltung kann eine möglichst große Oberfläche der Stimulationselektrode bereit gestellt werden, welche das Stimulationsverhalten der Stimulationselektrode verbessern kann.

Grundsätzlich ist denkbar, dass das Fluid am distalen Ende des Schlauchs zwischen der Stimulationselektrode und der Schlauchwandung austreten kann. Vorteilhafterweise ist die Stimulationselektrode mit einem Befestigungsabschnitt in dem distalen Ende des Schlauchs formschlüssig angeordnet, wobei der Schlauch in seiner Wandung wenigstens eine in den Spalt zwischen Stimulationsdraht und Schlauch mündende Zuspritzöffnung aufweist und/oder die Stimulationselektrode eine axiale Durchgangsöffnung aufweist. Die formschlüssige Anordnung ermöglicht eine Stabilisierung des distalen Endes des Stimulationsdrahts innerhalb des Schlauchs. Weiterhin kann die formschlüssige Anordnung einen Verschluss des distalen Endes des Schlauchs ermöglichen, um ein Eindringen von Partikeln in das distale Ende des Schlauchs oder gar ein Zusetzen des distalen Endes des Schlauchs zu verhindern. Das Fluid kann in dieser Ausführungsform seitlich durch die wenigstens eine in der Wandung des Schlauchs angeordnete Zuspritzöffnung und/oder durch die axiale Durchgangsöffnung der Stimulationselektrode austreten.

Vorzugsweise sind das proximale Ende des Schlauchs mit der Verbindungsstelle zu dem Stimulationskabel und der Verbindungsstelle zu dem Zuspritzschlauch in einem Gehäuse angeordnet, wobei das Stimulationskabel und der Zuspritzschlauch proximal, insbesondere parallel zur Längsachse des Schlauchs, aus dem Gehäuse austreten. Das proximale Ende des Schlauchs einschließlich der Verbindungsstellen kann dabei in das Gehäuse eingelegt und zusätzlich mit einem Kleber vergossen oder mit einem Gehäuse umspritzt werden. Auf diese Weise können die Verbindungsstellen geschützt angeordnet werden.

Der Durchmesser des Stimulationsdrahts beträgt vorzugsweise weniger als 0,3 mm, vorzugsweise etwa 0,2 mm. Auf diese Weise kann ein dünner, flexibler Stimulationsdraht bereitgestellt werden, der ein einfaches Vorschieben in den Körper, insbesondere in natürliche Körperöffnungen, ermöglichen kann.

Vorzugsweise ist der Stimulationsdraht aus Edelstahl gefertigt. Dadurch kann die erforderliche Stabilität des Stimulationsdrahts erreicht werden, insbesondere auch bei Längen von mehr als 10 cm.

Gemäß einer besonders bevorzugten Ausführungsform der Erfindung beträgt der Außendurchmesser des Schlauchs weniger als 1,0 mm, vorzugsweise weniger als 0,9 mm. Dadurch kann eine Dimensionierung der Vorrichtung zur Nervenstimulation ermöglicht werden, welche ein einfaches Einführen in den Körper, insbesondere in natürliche Körperöffnungen, ermöglichen kann.

Vorzugsweise liegt der Innendurchmesser des Schlauchs im Bereich von 0,4 mm bis 0,7 mm, vorzugsweise im Bereich von 0,5 mm bis 0,6 mm. Eine derartige Dimensionierung ermöglicht einen ausreichend großen Spalt zwischen Innenwandung des Schlauchs und Stimulationsdraht, um ein Fluid durch den Schlauch vom proximalen bis zum distalen Ende führen zu können.

Gemäß einer bevorzugten Weiterbildung der Erfindung beträgt die Länge des Schlauchs mehr als 10 cm, vorzugsweise mehr als 15 cm, besonders bevorzugt mehr als 20 cm, beispielsweise 32,2 cm. Dadurch wird eine genügende Länge bereitgestellt, um die Vorrichtung zur Nervenstimulation ausreichend weit in den Körper, beispielsweise in natürliche Körperöffnungen, einschieben zu können.

Eine bevorzugte Ausführungsform der Erfindung sieht vor, dass der Schlauch aus Kunststoff, beispielsweise einem Polyamid, gefertigt ist. Ein derartiges Material kann eine ausreichend Dichtigkeit, eine hohe Flexibilität sowie eine gute Biokompatibilität ermöglichen.

Vorzugsweise weist der Schlauch ausgehend von dem distalen Ende Markierungen auf, welche einem Benutzer visualisieren können, um welche Länge der Schlauch bereits in den Körper eingeschoben wurde.

Eine erfindungsgemäße Vorrichtung wird erfindungsgemäß zur Nervenstimulation durch eine natürliche Körperöffnung, beispielsweise die Nase, die Blase oder die Vagina, verwendet. Beispielsweise kann bei Einführen durch die Nase eine Nervenstimulation des Ganglion sphenopalatinum oder auch bei Zuspritzen eines Anästhetikums eine Blockade des Ganglion sphenopalatinums durchgeführt werden, um beispielsweise Gesichts- und Kopfschmerzen bei Migräne behandeln zu können.

Ein Ausführungsbeispiel der Erfindung wird anhand der nachfolgenden Figuren ausführlich erläutert. Es zeigen
- Fig. 1: eine Seitenansicht eines Ausführungsbeispiels einer erfindungsgemäßen Vorrichtung zur Nervenstimulation,
- Fig. 2: eine Detailansicht des distalen Endes der Vorrichtung gemäß Fig. 1,
- Fig. 3: einen Längsschnitt der distalen Endes gemäß Fig. 2,
- Fig. 4: eine teilweise geschnittene Ansicht der Vorrichtung gemäß Fig. 1 und
- Fig. 5: eine Ausschnittvergrößerung aus Fig. 4.

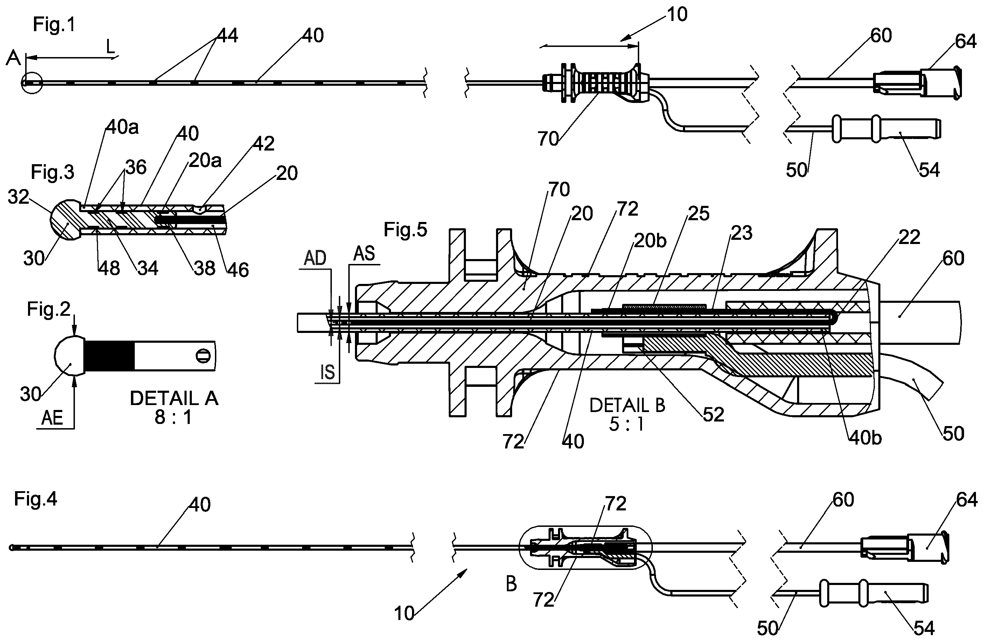

Die Figuren 1 bis 5 zeigen verschiedene Ansichten eines Ausführungsbeispiels einer Vorrichtung 10 zur Nervenstimulation. Zur besseren Übersicht sind nicht sämtliche Bezugsziffern in sämtlichen Figuren angegeben.

Die Vorrichtung 10 umfasst eine elektrisch leitenden Stimulationsdraht 20 mit einem Durchmesser AD, einem distalen Ende 20a und einem proximalen Ende 20b. Bei dem Stimulationsdraht 20 handelt es sich insbesondere um einen flexiblen, biegbaren Draht. Der Stimulationsdraht 20 ist beispielsweise aus Edelstahl gefertigt. Der Durchmesser AD des Stimulationsdrahts 20 kann weniger als 0,3 mm, vorzugsweise etwa 0,2 mm, betragen.

Weiterhin umfasst die Vorrichtung 10 einem elektrisch isolierenden Schlauch 40 mit einem Innendurchmesser IS, einem Außendurchmesser AS, einem distalen Ende 40a und einem proximalen Ende 40b. Bei dem Schlauch 40 handelt es sich insbesondere um einen flexiblen, dichten und biokompatiblen Schlauch 40. Der Schlauch 40 kann beispielsweise aus Kunststoff, vorzugsweise einem Polyamid, gefertigt sein. Der Außendurchmesser AS des Schlauchs 40 beträgt vorzugsweise weniger als 1,0 mm, besonders bevorzugt weniger als 0,9 mm, und kann beispielsweise 0,87 mm betragen. Der Innendurchmesser IS des Schlauchs 40 ist größer als der Durchmesser AD des Stimulationsdrahts 20 und kann im Bereich von 0,4 mm bis 0,7 mm, vorzugsweise im Bereich von 0,5 mm bis 0,6 mm, liegen und beispielsweise 0,54 mm betragen. Die Länge L des Schlauchs 40 beträgt mehr als 10 cm, vorzugsweise mehr als 15 cm, besonders bevorzugt mehr als 20 cm, beispielsweise 32,2 cm. Ausgehend von dem distalen Ende 40a kann der Schlauch 40 Markierungen 44 aufweisen (siehe Fig. 1 und 4), welche insbesondere äquidistant angeordnet sind und dem Benutzer Informationen darüber geben können, um welche Länge das distale Ende 40a des Schlauchs 40 in einen Körper eingeführt wurde.

Der Stimulationsdraht 20 ist in dem Schlauch 40 angeordnet, insbesondere durch das Lumen des Schlauchs 40 geführt. Dadurch, dass der Innendurchmesser IS des Schlauchs 40 größer ist als der Durchmesser AD des Stimulationsdrahts 20, wird dabei ein Spalt 46 in dem Schlauch 40, insbesondere nach Art eines Ringspalts zwischen dem Stimulationsdraht 20 und der Innenwandung des Schlauchs 40, gebildet, durch welchen wie nachfolgend näher beschrieben Flüssigkeit vom proximalen Ende 40b des Schlauchs 40 zum distalen Ende 40a des Schlauchs 40 geführt werden kann.

An dem distalen Ende 20a des Stimulationsdrahts 20 ist eine Stimulationselektrode 30 elektrisch leitend angeordnet, welche über das distale Ende 40a des Schlauchs 40 vorsteht. Die Stimulationselektrode 30 kann eine abgerundete Außenfläche 32 aufweist und beispielsweise kalottenförmig, kugelförmig, ellipsoidförmig, pilzförmig, birnenförmig oder eiförmig oder in sonstiger Weise ballig ausgebildet sein. Durch die konvexe Ausgestaltung der Stimulationselektrode 30 weist diese in wenigstens einem Querschnitt einen größten Außendurchmesser AE auf. Dieser Außendurchmesser AE der Stimulationselektrode 30 ist größer als der Außendurchmesser AS des Schlauchs 40 (vgl. insbesondere Fig. 2 und 3).

Die elektrisch leitende Verbindung zwischen dem Stimulationsdraht 20 und der Stimulationselektrode 30 kann beispielsweise dadurch gebildet sein, dass das distale Ende 20a des Stimulationsdrahts 20 in ein in der Stimulationselektrode 30 oder in einem an der Stimulationselektrode 30 angeordneten Befestigungsabschnitt 34 angeordnetes Sackloch 38 eingeführt und dort klemmend fixiert oder durch Verschweißen fixiert wird (siehe Figur 3).

Die Stimulationselektrode 30 kann mit dem Befestigungsabschnitt 34 in dem distalen Ende 40a des Schlauchs 40, genauer gesagt in einem von dem distalen Ende 40a ausgehenden Abschnitt 48 des Schlauchs 40, formschlüssig angeordnet sein. Der Befestigungsabschnitt 34 kann auf seiner Außenseite eine oder mehrere Rillen 36 aufweisen, welche eine klemmende Fixierung des Befestigungsabschnitts 34 in dem Abschnitt 48 des Schlauchs 40 verbessern. Die Stimulationselektrode 30, insbesondere der Befestigungsabschnitt 34, kann dadurch das distale Ende 40a des Schlauchs 40 verschließen. Um dennoch ein Austreten eines in dem Spalt 46 angeordneten Fluids am distalen Ende der Vorrichtung 10 ermöglichen zu können, kann der Schlauch 40 in seiner Wandung wenigstens eine in den Spalt 46 zwischen dem Stimulationsdraht 20 und dem Schlauch 40 mündende Zuspritzöffnung 42 (vgl. Fig. 3) und/oder die Stimulationselektrode 30 eine axiale Durchgangsöffnung (nicht dargestellt) aufweisen.

Das proximale Ende 20b des Stimulationsdrahts 20 steht über das proximale Ende 40b des Schlauchs 40 vor und ist, insbesondere in einem Biegeabschnitt 22, um das proximale Ende 40b des Schlauchs 40 umgebogen, insbesondere um etwa 180°, auf die Außenseite des Schlauchs 40 geführt. Ein elektrisch leitendes Stimulationskabel 50, insbesondere eine elektrisch leitende Ader 52 des Stimulationskabels 50, ist an dem proximalen Ende 20b des Stimulationsdrahts 20, welcher insbesondere mit einem sich an den Biegeabschnitt 22 anschließenden zurückgeführten Abschnitt 23 auf der Außenseite des Schlauchs 40 zu liegen kommt, elektrisch leitend verbunden. Die elektrisch leitende Kontaktierung des Stimulationsdrahts 20 kann somit außerhalb des bei Verwendung oft flüssigkeitsgefüllten Lumens des Schlauchs 40 und ohne Durchbruch durch die Wandung des Schlauchs 40 erfolgen. Das Stimulationskabel 50, insbesondere die Ader 52 des Stimulationskabels 50, kann beispielsweise mittels einer Crimphülse 25 elektrisch leitend mit dem Stimulationsdraht 20, insbesondere dem zurückgeführten Abschnitt 23, verbunden werden. Wie in den Figuren 1 und 4 dargestellt, kann am anderen Ende des Stimulationskabels 50 ein elektrischer Steckanschluss 54 zum Anschluss eines Steuergeräts mit Pulsgenerator angeordnet sein.

Ein Zuspritzschlauch 60 ist abschnittsweise über das proximale Ende 40b des Schlauchs 40, und dabei insbesondere abschnittsweise auch über den an der Außenseite des Schlauchs 40 liegenden zurückgeführten Abschnitt 23 des Stimulationsdrahts 20, abdichtend gestülpt. Dies kann beispielsweise dadurch erreicht werden, dass ein Innendurchmesser des Zuspritzschlauchs 60 in etwa dem Außendurchmesser AS des Schlauchs 40 entspricht. Wie in den Figuren 1 und 4 dargestellt, kann am anderen Ende des Zuspritzschlauchs 60 ein Spritzenanschluss 64 angeordnet sein, über welchen beispielsweise mit einer Spritze ein Fluid, beispielsweise ein Anästhetikum, in den Zuspritzschlauch 60 eingespritzt werden kann, welches über den Zuspritzschlauch 60 in das proximale Ende 40b des Schlauchs 40 eintritt und am distalen Ende 40a des Schlauchs 40, insbesondere durch die Zuspritzöffnung 42, austritt.

Das proximale Ende 40b des Schlauchs 40 mit der Verbindungsstelle zu dem Stimulationskabel 50 und der Verbindungsstelle zu dem Zuspritzschlauch 60 kann in einem Gehäuse 70 angeordnet sein, wobei das Stimulationskabel 50 und der Zuspritzschlauch 60 proximal, insbesondere parallel zur Längsachse des Schlauchs 40, aus dem Gehäuse 70 austreten. Das proximale Ende 40b des Schlauchs 40 einschließlich der Verbindungsstellen kann dabei in das Gehäuse 70 eingelegt und zusätzlich mit einem Kleber vergossen oder alternativ mit dem Gehäuse 70 umspritzt werden. Auf diese Weise können die Verbindungsstellen geschützt angeordnet werden. An dem Gehäuse 70 können ein oder mehrere Griffmulden 72 zum Einlegen der Finger eines Benutzers angeordnet sein, um die Griffsicherheit zu erhöhen.

Eine Vorrichtung 10 kann zur Nervenstimulation durch eine natürliche Körperöffnung, beispielsweise die Nase, die Blase **o**der die Vagina, verwendet werden. Beispielsweise kann bei Einführen durch die Nase eine Nervenstimulation des Ganglion sphenopalatinum mit der Stimulationselektrode 30 bei Anlegen beispielsweise von niederfrequentem Wechselstrom an den Stimulationsdraht 20 oder auch bei Zuspritzen eines Anästhetikums durch den Zuspritzschlauch 60 und den Schlauch 40 eine Blockade des Ganglion sphenopalatinums durchgeführt werden, um beispielsweise Gesichts- und Kopfschmerzen bei Migräne behandeln zu können.

### Bezugszeichenliste

- 10: Vorrichtung
- 20: Stimulationsdraht
- 20a: distales Ende
- 20b: proximales Ende
- 22: Biegeabschnitt
- 23: zurückgeführter Abschnitt
- 25: Crimphülse
- 30: Stimulationselektrode
- 32: Außenfläche
- 34: Befestigungsabschnitt
- 36: Rille
- 38: Sackloch
- 40: Schlauch
- 40a: distales Ende
- 40b: proximales Ende
- 42: Zuspritzöffnung
- 44: Markierung
- 46: Spalt
- 48: Abschnitt
- 50: Stimulationskabel
- 52: Ader
- 54: elektrischer Steckanschluss
- 60: Zuspritzschlauch
- 64: Spritzenanschluss
- 70: Gehäuse
- 72: Griffmulde
- AD: Durchmesser des Stimulationsdrahts
- AS: Außendurchmesser des Schlauchs
- IS: Innendurchmesser des Schlauchs
- AE: Außendurchmesser der Stimulationselektrode
- L: Länge

## Patentansprüche

1. Vorrichtung (10) zur Nervenstimulation mit einem elektrisch leitenden Stimulationsdraht (20) mit einem Durchmesser (AD), einem distalen Ende (20a) und einem proximalen Ende (20b), welcher in einem elektrisch isolierenden Schlauch (40) mit einem Innendurchmesser (IS), einem Außendurchmesser (AS), einem distalen Ende (40a) und einem proximalen Ende (40b) angeordnet ist, wobei der Innendurchmesser (IS) des Schlauchs (40) größer ist als der Durchmesser (AD) des Stimulationsdrahts (20), so dass ein Spalt (46) zur Zufuhr von Fluid in dem Schlauch (40) gebildet ist,
**dadurch gekennzeichnet, dass** an dem distalen Ende (20a) des Stimulationsdrahts (20) eine Stimulationselektrode (30) elektrisch leitend angeordnet ist, welche über das distale (40a) Ende des Schlauchs (40) vorsteht, und wobei das proximale Ende (20b) des Stimulationsdrahts (20) über das proximale Ende (40b) des Schlauchs (40) vorsteht und um das proximale Ende (40b) des Schlauchs (40) umgebogen auf die Außenseite des Schlauchs (40) geführt ist, wobei ein elektrisch leitendes Stimulationskabel (50) an dem proximalen Ende (20b) des Stimulationsdrahts (20) elektrisch leitend verbunden ist und wobei ein Zuspritzschlauch (60) abschnittsweise über das proximale Ende (40b) des Schlauchs (40) abdichtend gestülpt ist.

2. Vorrichtung nach einem der vorhergehenden Ansprüche, **dadurch gekennzeichnet, dass** die Stimulationselektrode (30) eine abgerundete Außenfläche (32) aufweist und beispielsweise kalottenförmig, kugelförmig, ellipsoidförmig, pilzförmig, birnenförmig oder eiförmig ausgebildet ist.

3. Vorrichtung nach einem der vorhergehenden Ansprüche, **dadurch gekennzeichnet, dass** die Stimulationselektrode (30) zumindest in einem Querschnitt einen Außendurchmesser (AE) aufweist, welcher größer ist als der Außendurchmesser (AS) des Schlauchs (40).

4. Vorrichtung nach einem der vorhergehenden Ansprüche, **dadurch gekennzeichnet, dass** die Stimulationselektrode (30) mit einem Befestigungsabschnitt (34) in dem distalen Ende (40a) des Schlauchs (40) formschlüssig angeordnet ist, wobei der Schlauch (40) in seiner Wandung wenigstens eine in den Spalt (46) zwischen Stimulationsdraht (20) und Schlauch (40) mündende Zuspritzöffnung (42) und/oder die Stimulationselektrode (30) eine axiale Durchgangsöffnung aufweist.

5. Vorrichtung nach einem der vorhergehenden Ansprüche, **dadurch gekennzeichnet, dass** das proximale Ende (40b) des Schlauchs (40) mit der Verbindungsstelle zu dem Stimulationskabel (50) und der Verbindungsstelle zu dem Zuspritzschlauch (60) in einem Gehäuse (70) angeordnet sind, wobei das Stimulationskabel (50) und der Zuspritzschlauch (60) proximal, insbesondere parallel zur Längsachse des Schlauchs (40), aus dem Gehäuse (70) herausgeführt sind.

6. Vorrichtung nach einem der vorhergehenden Ansprüche, **dadurch gekennzeichnet, dass** der Durchmesser (AD) des Stimulationsdrahts (20) weniger als 0,3 mm, vorzugsweise etwa 0,2 mm, beträgt.

7. Vorrichtung nach einem der vorhergehenden Ansprüche, **dadurch gekennzeichnet, dass** der Stimulationsdraht (20) aus Edelstahl gefertigt ist.

8. Vorrichtung nach einem der vorhergehenden Ansprüche, **dadurch gekennzeichnet, dass** der Außendurchmesser (AS) des Schlauchs (40) weniger als 1,0 mm, vorzugsweise weniger als 0,9 mm beträgt.

9. Vorrichtung nach einem der vorhergehenden Ansprüche, **dadurch gekennzeichnet, dass** der Innendurchmesser (IS) des Schlauchs (40) im Bereich von 0,4 mm bis 0,7 mm, vorzugsweise im Bereich von 0,5 mm bis 0,6 mm, liegt.

10. Vorrichtung nach einem der vorhergehenden Ansprüche, **dadurch gekennzeichnet, dass** die Länge (L) des Schlauchs (40) mehr als 10 cm, vorzugsweise mehr als 15 cm, besonders bevorzugt mehr als 20 cm, beträgt.

11. Vorrichtung nach einem der vorhergehenden Ansprüche, **dadurch gekennzeichnet, dass** der Schlauch (40) aus Kunststoff, beispielsweise einem Polyamid, gefertigt ist.

12. Vorrichtung nach einem der vorhergehenden Ansprüche, **dadurch gekennzeichnet, dass** der Schlauch (40) ausgehend von dem distalen Ende (40a) Markierungen (44) aufweist.

13. Vorrichtung nach einem der vorhergehenden Ansprüche, **dadurch gekennzeichnet, dass** die Vorrichtung zur Nervenstimulation durch eine natürliche Körperöffnung geeignet ist.

## Claims

1. Device (10) for nerve stimulation with an electrically conductive stimulation wire (20) with a diameter (AD), a distal end (20a) and a proximal end (20b), which is arranged in an electrically insulating tube (40) having an inside diameter (IS), an outside diameter (AS), a distal end (40a) and a proximal end (40b), wherein the inside diameter (IS) of the tube (40) is larger than the diameter (AD) of the stimulation wire (20), such that a gap (46) is formed for supplying fluid into the tube (40),
**characterised in that** a stimulation electrode (30) is electrically conductively arranged at the distal end (20a) of the stimulation wire (20), which electrode protrudes beyond the distal (40a) end of the tube (40), and wherein the proximal end (20b) of the stimulation wire (20) protrudes beyond the proximal end (40b) of the tube (40) and is guided around the proximal end (40b) of the tube in a manner bent around onto the outside of the tube (40), wherein an electrically conductive stimulation electrode (50) is electrically conductively connected to the proximal end (20b) of the stimulation wire (20) and wherein an injection tube (60) is pulled in a sealing manner in portions over the proximal end (40b) of the tube (40).

2. Device according to any of the preceding claims, **characterised in that** the stimulation electrode (30) has a rounded outer surface (32) and is configured to be for example dome-shaped, spherical, ellipsoidal, mushroom-shaped, pear-shaped or egg-shaped.

3. Device according to either of the preceding claims, **characterised in that** the stimulation electrode (30) has an outside diameter (AE), at least in a cross-section, that is larger than the outside diameter (AS) of the tube (40).

4. Device according to any of the preceding claims, **characterised in that** the stimulation electrode (30) is arranged in a form-fitting manner, with a fastening portion (34), in the distal end (40a) of the tube (40), wherein the tube (40) comprises, in its wall, at least one injection opening (42) that leads into the gap (46) between the stimulation wire (20) and tube (40), and/or the stimulation electrode (30) comprises an axial through-opening.

5. Device according to any of the preceding claims, **characterised in that** the proximal end (40b) of the tube (40) is arranged with the connection point to the stimulation cable (50) and the connection point to the injection tube (60) in a housing (70), wherein the stimulation cable (50) and the injection tube (60) are guided out of the housing (70) proximally, in particular in parallel with the longitudinal axis of the tube (40).

6. Device according to any of the preceding claims, **characterised in that** the diameter (AD) of the stimulation wire (20) is less than 0.3 mm, preferably approximately 0.2 mm.

7. Device according to any of the preceding claims, **characterised in that** the stimulation wire (20) is produced from stainless steel.

8. Device according to any of the preceding claims, **characterised in that** the outside diameter (AS) of the tube (40) is less than 1.0 mm, preferably less than 0.9 mm.

9. Device according to any of the preceding claims, **characterised in that** the inside diameter (IS) of the tube (40) is in the range of 0.4 mm to 0.7 mm, preferably in the range of 0.5 mm to 0.6 mm.

10. Device according to any of the preceding claims, **characterised in that** the length (L) of the tube (40) is more than 10 cm, preferably more than 15 cm, particularly preferably more than 20 cm.

11. Device according to any of the preceding claims, **characterised in that** the tube (40) is produced from plastics material, for example a polyamide.

12. Device according to any of the preceding claims, **characterised in that** the tube (40) comprises markings (44) proceeding from the distal end (40a).

13. Device according to any of the preceding claims, **characterised in that** the device is suitable for nerve stimulation through a natural body opening.

## Revendications

1. Dispositif (10) de stimulation nerveuse comprenant un fil (20) de stimulation conducteur de l'électricité ayant un diamètre (AD), un bout (20a) distal et un bout (20b) proximal, lequel est disposé dans un tuyau (40) souple, isolant électriquement, ayant un diamètre (IS) intérieur, un diamètre (AS) extérieur, un bout (40a) distal et un bout (40b) proximal, dans lequel le diamètre (IS) intérieur du tuyau (40) souple est plus grand que le diamètre (AD) du fil (20) de stimulation de manière à former un intervalle (46) pour l'envoi de fluide dans le tuyau (40),
**caractérisé en ce qu'**au bout (20a) du fil (20) de stimulation est montée, de manière conductrice de l'électricité, une électrode (30) de stimulation, qui est en saillie du bout distal (40a) du tuyau (40) souple, et dans lequel le bout (20b) proximal du fil (20) de stimulation est en saillie du bout (40b) proximal du tuyau (40) souple et passe sur la face extérieure du tuyau (40) souple en étant recourbé autour du bout (40b) proximal du tuyau (40) souple, dans lequel un câble (50) de stimulation conducteur de l'électricité est connecté d'une manière conductrice de l'électricité au bout (20b) proximal du fil (20) de stimulation et dans lequel un tuyau (60) souple de pulvérisation est retroussé d'une manière étanche par endroit sur le bout (40b) proximal du tuyau (40) souple.

2. Dispositif suivant l'une des revendications précédentes, **caractérisé en ce que** l'électrode (30) de stimulation a une surface (32) extérieure, arrondie et est, par ailleurs, constituée sous la forme d'une calotte, d'un ellipsoïde, d'un champignon, d'une poire ou d'un œuf.

3. Dispositif suivant l'une des revendications précédentes, **caractérisé en ce que** l'électrode (30) de stimulation a, au moins dans une section transversale, un diamètre (AE) extérieur, qui est plus grand que le diamètre (AS) extérieur du tuyau (40) souple.

4. Dispositif suivant l'une des revendications précédentes, **caractérisé en ce que** l'électrode (30) de stimulation est montée, par un tronçon (34) de fixation, à complémentarité de forme dans le bout (40a) distal du tuyau (40) souple, dans lequel le tuyau (40) souple a, dans sa paroi, au moins une ouverture (42) de pulvérisation débouchant dans l'intervalle (46) entre le fil (20) de stimulation et le tuyau (40) souple et/ou l'électrode (30) de stimulation a une ouverture axiale de passage.

5. Dispositif suivant l'une des revendications précédentes, **caractérisé en ce que** le bout (40b) proximal du tuyau (40) souple sont, avec le point de connexion au câble (50) de stimulation et le point de connexion avec le tuyau (60) souple de pulvérisation, disposés dans un boîtier (70), dans lequel le câble (50) de stimulation et le tuyau (60) souple de pulvérisation sortent du boitier (70) proximalement, en particulier parallèlement à l'axe longitudinal du tuyau (40) souple.

6. Dispositif suivant l'une des revendications précédentes, **caractérisé en ce que** le diamètre (AD) du fil (20) de stimulation est de moins de 0,3 mm, en étant de préférence d'environ 0,2 mm.

7. Dispositif suivant l'une des revendications précédentes, **caractérisé en ce que** le fil (20) de stimulation est en acier fin.

8. Dispositif suivant l'une des revendications précédentes, **caractérisé en ce que** le diamètre (AS) extérieur du tuyau (40) souple est inférieur à 1,0 mm, en étant de préférence inférieur à 0,9 mm.

9. Dispositif suivant l'une des revendications précédentes, **caractérisé en ce que** le diamètre (IS) intérieur du tuyau (40) souple est dans la plage allant de 0,4 mm à 0,7 mm, de préférence dans la plage allant de 0,5 mm à 0,6 mm.

10. Dispositif suivant l'une des revendications précédentes, **caractérisé en ce que** la longueur (L) du tuyau (40) souple est de plus de 10 cm, de préférence de plus de 15 cm, d'une manière particulièrement préférée de plus de 20 cm.

11. Dispositif suivant l'une des revendications précédentes, **caractérisé en ce que** le tuyau (40) souple est en matière plastique, par exemple en polyamide.

12. Dispositif suivant l'une des revendications précédentes, **caractérisé en ce que** le tuyau (40) souple a des repères (44) à partir du bout (40a) distal.

13. Dispositif suivant l'une des revendications précédentes, **caractérisé en ce que** le dispositif de stimulation nerveuse est propre à passer dans une ouverture naturelle du corps.
